# EUROPEAN PATENT SPECIFICATION

(11) **EP 2 167 566 B1**
(45) Date of publication and mention of the grant of the patent: **16.05.2012**
(21) Application number: 08786113.4
(22) Date of filing: 14.07.2008
(51) Int. Cl.: C08G 64/00, C08G 64/30

(54) **METHOD FOR STORAGE AND/OR TRANSPORT OF BISPHENOLACETONE AND METHOD FOR PRODUCING AROMATIC POLYCARBONATE**
VERFAHREN ZUM LAGERN UND/ODER TRANSPORTIEREN VON BISPHENOLACETON UND VERFAHREN ZUR HERSTELLUNG EINES AROMATISCHEN POLYCARBONATS
PROCÉDÉ DE STOCKAGE ET/OU DE TRANSPORT DE BISPHÉNOLACÉTONE ET PROCÉDÉ DE PRODUCTION DE POLYCARBONATE AROMATIQUE

(30) Priority: 18.07.2007 EP 07112681
(43) Date of publication of application: 31.03.2010
(73) Proprietor: Shell Internationale Research Maatschappij B.V., 2596 HR The Hague (NL)
(72) Inventor: VAPORCIYAN, Garo Garbis, Houston, Texas 77079 (US)
(74) Representative: Matthezing, Robert Maarten
(86) International application number: PCT/EP2008/059156
(87) International publication number: WO 2009/010486

(56) References cited:
- EP-A- 1 331 235
- WO-A-2005/026235
- US-A- 4 492 807
- US-A- 5 502 016
- US-A1- 2003 181 768
- US-B1- 6 590 128

## Description

The present invention relates to a method for storage and/or transport of bisphenolacetone (BPA or bisphenol A), i.e. 2,2-bis(4-hydroxyphenyl)propane. Further, the invention relates to a method for producing aromatic polycarbonate.

Bisphenolacetone is widely used as a starting material in the production of aromatic polycarbonate, hereinafter sometimes referred to simply as polycarbonate. Polycarbonate is a widely used raw material in many different manufacturing sectors. Due to the high hardness as well as good transparency of the material, it can be applied in applications as diverse as automotive windows and optical lenses. The demand for polycarbonate is believed to increase largely within the next years, which will require the production of polycarbonate to be improved in terms of efficiency and environmental impact.

One of the known processes for producing polycarbonates is based on the transesterification of bisphenolacetone with diarylcarbonate. The use of a diarylcarbonate, in particular diphenylcarbonate (DPC), has the advantage that phenol is liberated in the reaction of the diphenylcarbonate with bisphenolacetone to form polycarbonate. This phenol may in turn be recycled to the production of bisphenolacetone or diphenylcarbonate, for which it is a main raw material.

In a production process of a polycarbonate employing bisphenolacetone as a starting material, in order to hold the bisphenolacetone in a molten state, a temperature higher than 160 °C has to be employed. Bisphenolacetone has a high melting point of 158 °C. However, when bisphenolacetone is held in a molten state at a high temperature for several hours, it starts being colored, and if it is used as a starting material for making polycarbonate, the color tone of the obtained product tends to be impaired, and the product cannot be used as an ordinary polycarbonate product. Accordingly, bisphenolacetone has conventionally been held as a solidified powder as a single substance.

However, the solidified bisphenolacetone has to be handled as a powder, and various influences have been found such that the powder tends to easily block for example a piping of an apparatus, or dissolution in for example an aqueous alkali solution may be impaired. Accordingly, it has been devised to maintain a prill shape which is less likely to cause blocking and which is easily dissolved. Nevertheless it is difficult to continue a continuous operation for one year, and the operation has to be stopped every few months for washing the apparatus such as a conveyer, thus leading to a considerable production loss.

The handling and transport of solid bisphenolacetone have drawbacks common to handling of solids in general. For instance, the solid particles have to have a suitable size and size distribution according to their subsequent application, as otherwise the material may not flow freely due to blocking. This size and size distribution are difficult to maintain, as the particles are prone to sinter upon exposure to even moderately elevated temperature and/or pressure. The particles may also build-up electrostatic charges upon handling, which increases the hazard of explosions and fire. Additionally, contamination with dust during cooling, crushing or transport is difficult to avoid. This may lead to contamination of the polycarbonate, which is detrimental to properties of polycarbonate products, in particular when used in optical devices.

The best means to solve the problem is to subject bisphenolacetone to polymerization while holding it in a molten state without solidifying it. However, as mentioned above, the bisphenolacetone has a high melting point (i.e. as a single substance), and a higher temperature has to be employed so as to hold it in a molten state. However, when it is held in a molten state at a high temperature, 4-isopropenyl phenol (compound of the following Formula (1)) forms in several hours, bisphenolacetone starts being colored, and a polycarbonate produced employing it as a starting material has an impaired color tone and does not satisfy essentialities as a product. Accordingly, improvement of heat stability of bisphenolacetone as a polycarbonate starting material has strongly been desired.

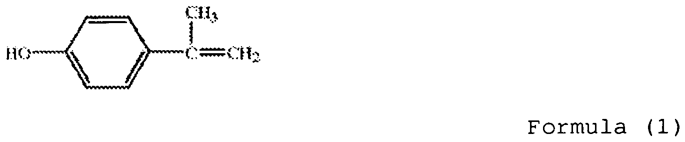

US 6,750,314 B2 discloses that in order to improve heat stability of bisphenolacetone in a molten state, it is most effective to decrease the holding temperature, i.e. to lower the melting point of bisphenolacetone. More in particular, US 6,750,314 B2 discloses that a composition comprising bisphenolacetone and phenol has a lower melting temperature than that of bisphenolacetone as a single substance. According to US 6,750,314 B2, an adduct crystal comprising bisphenolacetone and phenol in a weight ratio of 7/3 has a melting temperature of 120 °C.

Further, US 6,750,314 B2 discloses a process for producing a polycarbonate wherein such bisphenolacetone/ phenol composition is used, comprising:
(1) a step of reacting phenol with acetone in the presence of an acidic catalyst to convert part of the phenol into bisphenolacetone, to obtain a bisphenolacetone/phenol composition,
(2) a step of supplying the bisphenolacetone/phenol composition held in a molten state in a liquid form to a polycarbonate production step, and
(3) a step of subjecting the bisphenolacetone and a carbonate material to polymerization to produce an aromatic polycarbonate.

A disadvantage of the use of phenol in lowering the melting point of bisphenolacetone, is that phenol itself has a melting point which lies above room temperature, namely 43 °C. Therefore, in order to make the bisphenolacetone/phenol composition as disclosed in US 6,750,314 B2, heating at a relatively high temperature is to be applied. This is further demonstrated in the Comparative Examples below.

In addition, it has been found that the thermal stability of bisphenolacetone when combined with phenol, is relatively low. This low stability concerns the thermal decomposition of bisphenolacetone into 4-isopropenyl phenol as discussed above. This is also further demonstrated in the Comparative Examples below.

Therefore, there was a need to both lower the melting point of bisphenolacetone together with a substantial reduction of energy requirements, i.e. the energy required for conventional cooling for solidification and heating for melting of bisphenolacetone, and to further improve the thermal stability of bisphenolacetone.

Further, all of the polycarbonate production processes have in common that large amounts of separate raw materials need to be produced, transported and stored, or that several large production units must be combined on a single production site, which is usually not feasible for environmental and economical reasons.

More in particular, a polycarbonate production process, which combines the production of bisphenolacetone and polycarbonate, needs as raw materials acetone, phenol and diphenylcarbonate. The latter two are solids at ambient temperature, which implies that if large amounts of these materials are transported, a number of problems arise that affect both the safety and the economics of the overall process.

Bisphenolacetone has a melting point of 158 °C and, as disclosed in US 6,750,314 B2, a composition comprising bisphenolacetone and phenol in a weight ratio of 7/3 has a melting temperature of 120 °C, which makes transport of either bisphenolacetone alone or in combination with phenol in molten state impracticable, as most standard transport vessels for liquid materials are not equipped to maintain a temperature above 70 °C. However, safe transport and handling of the molten product (e.g. with minimal waste from tank washings) requires maintaining the product at a temperature of about 15 to 20 °C above the melting point. Transport of liquid materials at such temperature would require a large amount of energy, and could lead to problems with solidifying material if not properly handled. Only a limited number of vessels are even capable of such proper handling at these temperatures, all with rather small tank sizes.

Further, the transport of bisphenolacetone in the solid state on the other hand requires the bisphenolacetone, optionally in combination with phenol, to be solidified after its production. This is usually accomplished by cooling the bisphenolacetone, optionally in combination with phenol, and by forming it into suitable particles, which can then be bagged and transported as solid material. Generally, cooling and particle formation require large and complicated equipment such as cooling bands and/or prill towers. Such equipment unnecessarily increases the capital investment, and is expensive and energy consuming to operate. Therefore, it is generally an object not to have to store and transport bisphenolacetone in the solid state.

The above-mentioned objects can be achieved by transporting bisphenolacetone to a polycarbonate production plant as a liquid mixture with acetone, rather than phenol as taught in US 6,750,314 B2. The polycarbonate produced by using such bisphenolacetone/ acetone liquid mixture does not contain any coloring originating from the bisphenolacetone used to produce it.

Accordingly, the present invention relates to a method for producing aromatic polycarbonate, including the steps of:
(1) transporting a liquid mixture of acetone and bisphenolacetone to a polycarbonate production plant;
(2) separating the bisphenolacetone from the acetone;
(3) reacting the bisphenolacetone with diphenylcarbonate to produce polycarbonate.

Preferably, the method of the present invention is an integrated method, including the additional steps of:
(4) reacting phenol liberated in step (3) with acetone, optionally acetone of step (2), to produce bisphenolacetone;
(5) using the bisphenolacetone of step (4) and acetone, optionally acetone of step (2), in making the liquid mixture of acetone and bisphenolacetone.

The acetone of step (2) may be reacted with phenol, optionally phenol liberated in step (3), to produce bisphenolacetone and/or may be used in combination with bisphenolacetone in making the liquid mixture of acetone and bisphenolacetone.

The subject method resides on the insight that the above disadvantages and problems with the transport of both molten and solid bisphenolacetone, either alone or in combination with phenol, can be avoided by dissolving bisphenolacetone in acetone. Just like phenol, acetone is also used in the manufacture of bisphenolacetone, which in turn is a raw material for polycarbonate. Theoretically, 1 mole of acetone is required together with two moles of phenol to form 1 mole of bisphenolacetone. In turn, 2 moles of phenol are liberated per mole of diphenylcarbonate reacted with bisphenolacetone to obtain a polycarbonate. Generally, the production of polycarbonate with an integrated production of bisphenolacetone requires about equimolar amounts of acetone and diphenylcarbonate to be present at the polycarbonate factory.

It has now been discovered that acetone is a suitable solvent for bisphenolacetone, providing the required viscosity reduction at a temperature range of 15 to 90 °C, which is generally considered as a suitable temperature range for transport and handling. Thus, preferably the temperature during step (1) of the method of the present invention is in the range of from 15 to 90 °C, more preferably 15 to 55 °C. It has further been found that bisphenolacetone dissolves in acetone in such way that at ambient or slightly elevated temperature, the amount of acetone required to dissolve the bisphenolacetone is in the molar range required for the manufacture of bisphenolacetone from the liberated phenol. Thus, by using acetone as the solvent for bisphenolacetone, the subject method permits to combine two important raw materials of the above process without creating the necessity for returning spent solvent. Furthermore, by this approach, the solidification of bisphenolacetone, either alone or in combination with phenol, and problems associated with the handling of the solid are avoided.

Additionally, the combination of two important raw materials into a liquid mixture, which otherwise were transported and stored in separate vessels and tanks, reduces the need for storage capacity during transport as well as at the customer site, as no separate acetone and bisphenolacetone tanks are required.

The exact ratio of acetone to bisphenolacetone in the liquid mixture will be determined by the requirements of transport and handling as well as by the amount required at the polycarbonate production site. Preferably, the molar ratio of acetone to bisphenolacetone in the liquid mixture is in the range of from 0.5:1 to 15:1. However, if there is another use for acetone at the polycarbonate production site, higher amounts of acetone may be employed for the liquid mixture. Further, if acetone is also used as a solvent for the diphenylcarbonate, as is disclosed in WO 2005/026235, lower amounts of acetone may be employed for the bisphenolacetone/acetone liquid mixture. Such joint use of acetone as a solvent introduces great flexibility in an integrated polycarbonate production process. Further, if acetone is used as a solvent for both the bisphenolacetone and the diphenylcarbonate both mixtures may be combined in an appropriate ratio and be subjected to acetone removal in one single unit prior to feeding of the resulting mixture of bisphenolacetone and diphenylcarbonate to the polycarbonate production unit where both components are finally reacted with each other. The use of acetone as the only solvent for both said reaction components would thus greatly simplify an integrated polycarbonate production process and make it more flexible.

Conveniently, the molar ratio of acetone to bisphenolacetone is at least in the molar range required to achieve a liquid mixture at the desired storage and transport temperature that allows easy handling, for instance about 3:1. At this ratio, the liquid mixture can be handled and stored at about 35 °C, a temperature usually considered as normal for the transport of liquids in tank transporters and tank ships. Such temperature is considerably below the temperature of 120 °C which is the melting temperature for a preferred composition comprising bisphenolacetone and phenol in a weight ratio of 7/3 as disclosed in US 6,750,314 B2. With respect to a stable bisphenolacetone/phenol composition and the holding temperature in holding bisphenolacetone in a molten state, US 6,750,314 B2 discloses that the more stable composition is from 7/3 to 4/6, and the holding temperature is from 130 to 100 °C.

In accordance with the present invention, it is also possible to have the molar ratio of acetone to bisphenolacetone being determined by the requirements for low viscosity during handling and storage of the liquid mixture. In order to achieve a suitably low viscosity, the molar ratio of acetone to diphenylcarbonate is preferably at least 0.5:1, more preferably at least 0.6:1, again more preferably at least 0.8:1 and yet more preferably at least 0.9:1. On the other hand, the molar ratio of acetone to bisphenolacetone in the liquid mixture preferably is at most 12:1, more preferably at most 10:1, yet more preferably at most 5:1, again more preferably at most 3.5:1, and most preferably at most 3:1 in order to achieve a sufficiently low viscosity and good balance between the required amounts of both components in the overall process.

The first step of transporting a liquid mixture of acetone and bisphenolacetone to a polycarbonate production plant in accordance with the method of the present invention, is preceded by a step of making such liquid mixture. Making a liquid mixture of acetone and bisphenolacetone can preferably be performed while the bisphenolacetone is still liquid at the bisphenolacetone production site, for instance by adding the acetone to a stirred vessel containing the liquid bisphenolacetone, or by adding the liquid bisphenolacetone to acetone, until the desired acetone/bisphenolacetone ratio is obtained. Thus, the hot freshly produced liquid bisphenolacetone is preferably immediately dissolved in the acetone, thereby avoiding storage of hot bisphenolacetone. The liquid mixture can then be stored or transported and shipped without, or with only moderate additional heating, which requires only the customary insulation of transport tanks and piping to avoid substantial heat loss.

Tests have revealed that upon extended storage at the storage temperature range, the bisphenolacetone and solution properties remain unchanged.

In accordance with the present invention, the bisphenolacetone and/or the diphenylcarbonate comprise the products of unsubstituted phenol, as well as those of substituted phenols. In particular, brominated phenols have been found to improve the fire resistance of polycarbonate. Accordingly, the bisphenolacetone and/or diphenylcarbonate used in the present invention may comprise the products of a substituted phenol, in particular of a brominated phenol.

In step (1) of the subject method, a liquid mixture of acetone and bisphenolacetone is transported to a polycarbonate production plant. This transport may be performed as commonly applied for liquid chemical products in a bulk transport. The term transport includes storing and moving in suitable transport vessels.

Suitable storage and transport vessels include vessels such as road and rail tankers, bulk containers, tank barges and tank ships, storage tanks, drums and pipelines.

The material of the transport vessels should of course be proof against the liquid mixture and the temperature used. Preferred material is stainless steel.

The storage temperature generally is in the range of from ambient temperature to about 100 °C and the transport temperature generally is in the range of from ambient temperature to about 70 °C, depending on the relative amounts of bisphenolacetone and acetone.

If the liquid mixture is transported at elevated temperature, the transport and storage vessels are customarily insulated to reduce heat loss, and equipped with the necessary safety devices required.

At the production site, the liquid mixture can be easily separated into bisphenolacetone and acetone, which bisphenolacetone can directly be employed at step (3) of the method of the present invention. The separated acetone may be reacted with phenol, optionally phenol liberated in step (3), to produce bisphenolacetone and/or may be used in combination with bisphenolacetone in making the liquid mixture of acetone and bisphenolacetone.

Acetone may be added or partly removed at any stage of the transport or handling, as required. For instance, excessive acetone may be removed during the loading of larger barges, and may be added again when the barge is unloaded in order to achieve a suitable transport viscosity.

In step (2), the bisphenolacetone is separated from the acetone in the liquid mixture. This separation can conveniently be performed by a distillation treatment. Such a distillation treatment may be performed by a flash unit, or in a continuous distillation column, where the acetone is removed as top product, while the bisphenolacetone remains in the bottom fraction. Alternatively, a continuous film evaporator unit may be employed to avoid prolonged exposure of the bisphenolacetone to elevated temperatures.

As compared to US 6,750,314 B2, it is easier to separate acetone, having a boiling point of only 57 °C, than phenol from bisphenolacetone, phenol having a boiling point of 182 °C. Thus it requires a lot of energy to separate phenol from the bisphenolacetone. In addition, at such a high temperature bisphenolacetone is prone to thermal degradation leading to unwanted side-products such as 4-isopropenyl phenol.

Further, the boiling points of acetone and bisphenolacetone are so far apart that the obtained acetone is sufficiently pure for further use without additional purification. However, if desired a separate purification unit, for instance a distillation column, may be added to further purify the acetone.

The bottom fraction in the distillation unit mainly consists of bisphenolacetone. It is however not required to remove all of the acetone. A small amount of acetone remaining in bisphenolacetone fraction even has beneficial effects in the following steps, while also permitting a reduction of the energy consumption in this separation step (2).

In step (3), the bisphenolacetone is reacted with diphenylcarbonate to produce polycarbonate, as for instance described in US-A-6,277,945. Diphenylcarbonate can be produced in a number of ways, including the phosgenation of phenol or the oxidative carbonylation of phenol, both of which involve cumbersome reaction steps and create unnecessary amounts of waste.

A process which is more effective due to a more integrated raw material cycle, includes the steps of (i) reacting an olefin (e.g. propylene) and an oxidant to obtain the corresponding alkylene oxide (e.g. propylene oxide), then (ii) reacting the obtained alkylene oxide with carbon dioxide to obtain a cyclic alkylene carbonate (e.g. propylene carbonate), then (iii) reacting the obtained cyclic alkylene carbonate with an alcohol (e.g. methanol, ethanol and isopropanol) to obtain a dialkylcarbonate and the corresponding alkylene glycol (e.g. propylene glycol), and (iv) reacting the obtained dialkylcarbonate with phenol to obtain diphenylcarbonate, for instance as described in US-A-5,543,546.

The polycarbonate produced in said step (3), may still contain some acetone. Depending on the end application of such polycarbonate, said residual acetone might have to be removed at a later stage. Normally, substantially all of the acetone is removed together with the phenol during and/or after the reaction for producing the polycarbonate. Phenol is a reaction product of such reaction, which has a K_{eq} constant of about 1. As compared to phenol which is used in the bisphenolacetone composition of US 6,750,314 B2, acetone can be separated more easily from polycarbonate, e.g. simply by flashing-off. For some end applications it is undesirable to have residual phenol in the final polycarbonate whereas the presence of some acetone (if any) is relatively harmless.

Further, even though in melt processes for polycarbonate production some residual phenol in bisphenolacetone can be tolerated, even a small residual amount of phenol influences the reaction of diphenylcarbonate and bisphenolacetone to melt-polycarbonate and phenol in that its presence favours the reverse reaction so that more reaction time is required to produce the polycarbonate. And, as melt-polycarbonate is a heat sensitive material, additional reaction time likely means additional degradation of the polycarbonate.

In said step (3) of the subject method, phenol is liberated. Under the reaction conditions, the liberated phenol can immediately be removed from the mixture, so that no additional removal step is required.

Further, in said step (3), a small amount of acetone, when present in the bisphenolacetone, helps to reduce the viscosity and melting point of bisphenolacetone, which improves the handling of the bisphenolacetone melt. As a result, the melt process can be performed at a lower temperature. On the other hand, some undesired side-reactions involving acetone may occur, which is however strongly dependent on the specific polymerization catalyst used.

The presence of a small amount of acetone also has the following beneficial effects on the overall reaction:

It improves the mixing of the components, which allows a better control of the molecular weight distribution of the polycarbonate product.

Additionally, the removed mixture containing phenol and acetone can directly be employed in the manufacture of bisphenolacetone without further purification.

Accordingly, the bisphenolacetone fraction obtained in step (2) and used in step (3) preferably still contains at most 3% by weight of acetone, more preferably at most 2%, again more preferably at most 1.5%, yet more preferably at most 1%, and most preferably at most 0.5%.

In optional step (4), the phenol of step (3) is reacted with acetone, optionally acetone of step (2), to produce bisphenolacetone.

In optional step (5), the thus obtained bisphenolacetone and acetone, optionally acetone of step (2), are used in making the liquid mixture of acetone and bisphenolacetone. This production and use of bisphenolacetone close the overall cycle of the process, which permits to achieve a hitherto unknown level of integration, thereby making such process highly energy efficient as well as making efficient use of all raw materials used, while also reducing the need for separate transport, handling and storage of the raw materials of the overall process.

Finally, the present invention relates to :
- use of a liquid mixture of acetone and bisphenolacetone in producing aromatic polycarbonate, wherein the molar ratio of acetone to bisphenolacetone in the liquid mixture is in the range of from 0.5:1 to 15:1;
- a method for storage and/or transport of bisphenolacetone for use in producing aromatic polycarbonate, which involves the addition of such amount of acetone to bisphenolacetone that the molar ratio of acetone to bisphenolacetone in the final liquid mixture to be stored and/or transported is in the range of from 0.5:1 to 15:1; and

The above-mentioned use in producing aromatic polycarbonate also includes the use in a process wherein the polycarbonate is produced from phosgene, rather than diphenylcarbonate, and bisphenolacetone.

### Examples and Comparative Examples

### A. Solubility experiments

0.6 gram of bisphenolacetone (BPA) powder was placed in a 10 ml graduated tube with a thread seal. Prior to the experiment, BPA flakes were crushed to said powder to ensure uniform packing density at the bottom of the tube. Then 0.2 gram of a solvent was added to the BPA. The solvent was either acetone (present invention) or phenol (comparative). Thus, the weight ratio of BPA to solvent was 7.5/2.5. The tube was then immersed in an oil bath at a certain temperature, and mixed up to full equilibrium.

The foregoing was repeated except that the temperature of the oil bath was set at different values. Table 1 mentions the amounts of BPA dissolved in either acetone or phenol at temperatures ranging from 30 to 110 °C. The amount of BPA dissolved was calculated from the loss of BPA from the solids layer at the bottom of the tube.

**Table 1**

| T (°C) | Vol. % of BPA dissolved in acetone | Vol. % of BPA dissolved in phenol |
|---|---|---|
| 30 | 61 | not determined |
| 60 | 72 | 0 |
| 75 | 72 | 0 |
| 90 | 74 | 0 |
| 110 | 74 | 61 |

From Table 1 it can be concluded that a higher temperature is required to dissolve bisphenolacetone in phenol than in acetone.

### B. Stability experiments

BPA and a solvent were mixed to produce solutions (containing no solids) having a weight ratio of BPA to solvent of 6:4. The solvent was either acetone (present invention) or phenol (comparative). Where the solvent was acetone the solution was obtained at a temperature of 35 °C and then kept at that temperature. However, in view of the above solubility experiments, where the solvent was phenol the solution could only be obtained at a temperature of 120 °C and was then kept at that temperature.

The solutions were heated for a certain time period (3, 7 or 14 days) at either 35 °C (acetone) or 120 °C (phenol). At the end of each time period, the amount of 4-isopropenyl phenol (IPP) was determined by means of GC-MS. Table 2 mentions the intensity of the GC-MS signal at m/z = 134, which signal is characteristic for IPP, relative to a standard BPA sample that had not been subjected to said temperature treatment.

**Table 2**

| Time (days) | IPP signal intensity where solvent is acetone | IPP signal intensity where solvent is phenol |
|---|---|---|
| 3 | 0.1 | 14.8 |
| 7 | 0.0 | 22.3 |
| 14 | 0.0 | 35.0 |

From Table 2 it can be concluded that where phenol is used as the solvent for BPA and the resulting solution is kept at 120 °C, BPA is thermally degraded into IPP. On the other hand, a solution of BPA in acetone can be made at 35 °C storage at which temperature does not result in any substantial thermal degradation of the BPA.

## Claims

1. A method for producing aromatic polycarbonate, including the steps of:
(1) transporting a liquid mixture of acetone and bisphenolacetone to a polycarbonate production plant;
(2) separating the bisphenolacetone from the acetone;
(3) reacting the bisphenolacetone with diphenylcarbonate to produce polycarbonate.

2. A method according to claim 1, wherein the molar ratio of acetone to bisphenolacetone in the liquid mixture transported in step (1) is in the range of from 0.5:1 to 15:1.

3. A method according to claim 1 or 2, wherein the temperature during step (1) is in the range of from 15 to 90 °C.

4. A method for storage and/or transport of bisphenolacetone for use in producing aromatic polycarbonate, which involves the addition of such amount of acetone to bisphenolacetone that the molar ratio of acetone to bisphenolacetone in the final liquid mixture to be stored and/or transported is in the range of from 0.5:1 to 15:1.

## Patentansprüche

1. Verfahren zur Herstellung von aromatischem Polycarbonat, umfassend die folgenden Schritte:
(1) Transportieren einer flüssigen Mischung aus Aceton und Bisphenolaceton zu einer Polycarbonat-Herstellungsanlage;
(2) Trennen des Bisphenolacetons vom Aceton;
(3) Reagierenlassen des Bishpenolacetons mit Diphenylcarbonat zur Herstellung von Polycarbonat.

2. Verfahren nach Anspruch 1, wobei das molare Verhältnis von Aceton zu Bisphenolaceton in der in Schritt (1) transportierten flüssigen Mischung im Bereich von 0,5:1 bis 15:1 liegt.

3. Verfahren nach Anspruch 1 oder 2, wobei die Temperatur in Schritt (1) im Bereich von 15 bis 90°C liegt.

4. Verfahren zur Lagerung und/oder zum Transport von Bisphenolaceton zur Verwendung bei der Herstellung von aromatischem Polycarbonat, das die Zugabe von Aceton zu Bishpenolaceton in einer solchen Menge umfasst, dass das Molverhältnis von Aceton zu Bishpenolaceton in dem fertigen, zu lagernden und/oder zu transportierenden flüssigen Gemisch im Bereich von 0,5:1 bis 15:1 liegt.

## Revendications

1. Procédé de production de polycarbonate aromatique, englobant les étapes consistant à :
(1) transporter un mélange liquide d'acétone et de bisphénolacétone à une installation de production de polycarbonate ;
(2) séparer le bisphénolacétone de l'acétone ;
(3) faire réagir le bisphénolacétone avec du diphénylcarbonate pour obtenir du polycarbonate.

2. Procédé selon la revendication 1, dans lequel le rapport molaire de l'acétone au bisphénolacétone dans le mélange liquide transporté à l'étape (1) se situe dans la plage de 0,5:1 à 15:1.

3. Procédé selon la revendication 1 ou 2, dans lequel la température au cours de l'étape (1) se situe dans la plage de 15 à 90 °C.

4. Procédé pour l'entreposage et/ou le transport de bisphénolacétone à utiliser dans la production de polycarbonate aromatique, qui implique l'addition d'une quantité d'acétone par rapport au bisphénolacétone telle que le rapport molaire de l'acétone au bisphénolacétone dans le mélange liquide final qui doit être entreposé et/ou qui doit être transporté se situe dans la plage de 0,5:1 à 15:1.
